# EUROPEAN PATENT SPECIFICATION

(11) **EP 2 278 953 B1**
(45) Date of publication and mention of the grant of the patent: **04.05.2016**
(21) Application number: 09767290.1
(22) Date of filing: 27.05.2009
(51) Int. Cl.: A61K 9/00, A61K 9/107, A61K 47/00, A61K 47/14, A61K 47/18, A61K 47/24

(54) **SELF-PRESERVED EMULSIONS**
SELBSTERHALTENDE EMULSIONEN
ÉMULSIONS À AUTO-PRÉSERVATION

(30) Priority: 28.05.2008 US 56675 P
(43) Date of publication of application: 02.02.2011
(73) Proprietor: Alcon Research, Ltd, Fort Worth, Texas 76134 (US)
(72) Inventor: CHOWHAN, Masood, A., Arlington TX 76016 (US); GHOSH, Malay, Fort Worth TX 76109 (US)
(74) Representative: Best, Michael
(86) International application number: PCT/US2009/045282
(87) International publication number: WO 2009/154978

(56) References cited:
- WO-A1-95/01414
- US-A- 6 120 758
- US-B1- 6 451 775

## Description

### Technical Field

The present invention relates to emulsions for topical or internal use, particularly for ocular, intraocular, otic, and/or nasal applications. The present invention also relates to methods of both preserving and stabilizing such emulsions. The emulsions are particularly intended for use in ophthalmic drug delivery, and may be useful to increase the drug concentration or to enhance the bioavailability of hydrophobic drugs. The emulsions of the present invention are typically stable, non-irritating to ocular tissues, meet international preservation standards or a combination thereof.

### Background

Although ophthalmic drugs are typically delivered to the eye in aqueous solutions, many drugs which are therapeutically useful are insufficiently soluble in an aqueous vehicle to be provided in solution form. Alternative dosage forms for these lipid-soluble agents include ointments, suspensions and emulsions. Emulsions, particularly of the oil in water type, provide a means to deliver hydrophobic drugs either to the ocular surface or intraocularly. Although use of emulsions has been limited based on their difficulty of formulation, challenges with preservation, and comfort and irritation issues, there has been increasing interest in emulsions and microemulsions for ophthalmic use.

Broadly, emulsions are blended mixtures of two or more normally immiscible substances, usually fluids. In forming an emulsion, one substance is dispersed in the other. For example, in forming an oil-in-water emulsion, oil (the dispersed phase) is dispersed into water (termed the continuous phase).

Emulsions do not form spontaneously. Emulsions require some form of energy input, for example, stirring, shaking or spraying, in order to be formed. Intensive mixing of this nature is sometimes termed homogenization. Emulsions are also typically unstable. Over time, emulsions tend to separate into the more stable state of separation, for example, oil and water. Surface active agents, termed emulsifiers in this context, can increase the kinetic stability of emulsions. Many physical and chemical methods are used to prepare and stabilize emulsions, for example by heating, homogenization, and adding thickening agents, to name a few. However, use of these methods can add to the complexity, time and expense of preparing emulsions.

Compositions, including emulsions, intended for pharmaceutical use typically include preservatives to prevent or retard microbial contamination. Choice of a particular preservative or biocidal agent is usually closely tied to the intended application area. For example, for topical ophthalmic use, preservation is frequently accomplished by using a biocidal agent selected from a limited number of chemical classes, based on their relative safety and efficacy. Ophthalmic biocides typically fall into one of the following chemical classes: biguanides, quaternary ammonium compounds, polyamines and amides.

Various methods of testing are in use in order to ensure the safety of such products. For example, the United States Pharmacopeia (USP), a compendium of quality control tests for drugs and excipients used in medicinal formulations includes an antimicrobial effectiveness test (AEP), based on products and their route of administration. In the USP AEP, category 2 applies to topically used products made with aqueous bases or vehicles, non-sterile nasal products, and emulsions, including those applied to mucous membranes. An alternative, in some ways more stringent, testing method is detailed in the European Pharmacopoeia (Ph. Eur.) and the British Pharmacopeia (BP).

According to the United States Pharmacopoeia (USP Chapter 1151), "all emulsions require an antimicrobial agent because the aqueous phase is favorable to the growth of microorganisms". It is often difficult to preserve emulsions, in part due to the partitioning of the antimicrobial agent from the aqueous phase.

Emulsions intended for pharmaceutical use must also demonstrate a measure of physical stability. That is, there should be little or no evidence of creaming or separation upon storage.

Emulsifiers, or emulsion stabilizers, are frequently used to stabilize the dispersion of oil. The interactions between the emulsifier and the composition can be complex, as the emulsifier may modify the interface, as well as the environments of either or both phases.

Thickening agents may also be used to stabilize emulsions. Various polymers are also used to stabilize emulsions, functioning either as thickening agents, or to form structured interfacial films to prevent coalescence.

Several prior references teach agents with antimicrobial or other properties. U.S. Patent Nos. 4,209,449 and 4,503,002 (Mayhew et al.) describe phosphate quaternary compounds. U.S. Patent No. 4,215,064 describes phosphobetaines. PCT Appl. No. 2006/029255 (Scholz) describe cationic antiseptic compositions. U.S. Patent No. 6,120,758 (Siddiqui et al.) describes preservatives systems for topically applied products.

### Summary Of The Invention

The invention releates to an emulsion for ocular, nasal or otic administration comprising water, a buffering agent, an oil, an emulsifier, a pH adjuster, a multifunctional synthetic compound of formula I disclosed in claim 1 and optionally a therapeutic agent, wherein the amount of the compound of formula I is from 0.05 to 0.2%.

Surprisingly, emulsions have now been discovered that exhibit a combination or all of the following characteristics: they do not require heating, homogenization or thickening agents for emulsion stabilization, they pass European Pharmacopoeial testing paradigms, they can be manufactured without elaborate or special equipment, and/or they do not require an added preservative. Furthermore, the emulsions of the present invention are typically not irritating to ocular tissues, nor are they uncomfortable upon topical ocular administration. Thus, the present invention relates to emulsions that are typically stable, comfortable, low-irritancy, self-preserved or any combination thereof. The emulsions of the present invention are useful, *inter alia,* for the delivery of therapeutic pharmaceuticals. Among other factors, the present invention is based upon the discovery that certain multifunctional synthetic compounds can uniquely perform the dual function of both stabilizing and preserving pharmaceutical emulsions, and can provide an emulsion that is comfortable and non-irritating and/or can be suitable, *inter alia,* for topical use, for example, to deliver therapeutic agents to eye, ear, or nasal passages or tissues.

In preferred embodiments, the emulsions of the present invention have sufficient preservation efficacy so that the emulsions pass one or more standard preservative efficacy tests, such as the United States Preservative Efficacy Test (USPET), the European Preservative Efficacy Test-A (EP-A), the European Preservative Efficacy Test-B (EP-B), and similar standard tests. Most preferably, the emulsions of the present invention are sufficiently preserved so that they are able to pass one or more of these tests without the inclusion of a conventional preserving agent.

The present invention is based in part on the finding that certain multifunctional synthetic compounds are capable of performing the dual function of acting both as a preserving agent for emulsion compositions and also as an emulsion stabilizer. The multifunctional synthetic compounds have a unique molecular arrangement wherein a phosphate group is linked to one, two or three quaternary ammonium functionalities via a substituted propenyl group, and each quaternary ammonium functionality is further linked to at least one hydrocarbon chain. Without wishing to be bound by theory, it is believed that it is this unique molecular arrangement that enables these compounds to impart desired properties, including preservation and stabilization, to the emulsions of the present invention. The present invention is based in part as well on the additional finding that the emulsion compositions stabilized and preserved with these multifunctional synthetic compounds are both comfortable and non-irritating to the eye. The emulsions of the present invention can be prepared without the use of heat, homogenization or thickening agents.

### Brief Description of Drawings

Figure 1A is a histogram of the initial emulsion particle size measurements for Formulation B.
Figure 1B is a histogram of Formulation B emulsion particle size measurements at 60 weeks.
Figure 2A is a histogram of the initial emulsion particle size measurements for Formulation C.
Figure 2B is a histogram of Formulation C emulsion particle size measurements at 62 weeks.

### Detailed Description Of The Invention

The present invention is directed to emulsions containing compounds of the formula: 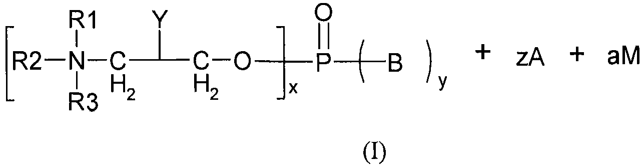 wherein:
x is 1 to 3 or mixtures thereof;
x+y is equal to 3;
a is 0 to 2;
z is equal to x;
Bis O- or OM;
A is an anion;
M is a cation;
Y is selected from the group consisting of OH, O-C₁-C₁₀ alkyl and O-C₁-C₁₀ alkenyl;
and
R1, R2 and R3 are the same or different and are alkyl, substituted alkyl, alkyl aryl or alkenyl groups, optionally interrupted by NHC(=O)-, of up to 16 carbon atoms with the proviso that the total carbon atoms in R1+R2+R3 is between 10 and 24.

In the foregoing definitions of R1, R2, R3, A, M and Y substituents, and throughout, the following terms unless otherwise indicated, shall be understood to have the following meanings:
The term "alkenyl" includes straight or branched chain hydrocarbon groups having 1 to 30 carbon atoms with at least one carbon-carbon double bond, the chain being optionally interrupted by one or more heteroatoms. The chain hydrogens may be substituted with other groups, such as, halo, -CF₃, -NO₂, -NH₂, -CN, -OCH₃, -C₆H₅,-C₆H₅O-alkyl, -O-C₆H₅O-alkenyl, p-NHC(=O)-C₆H₅-NHC(=O)-CH₃, -CH=NH, - NHC(=O)-Ph and -SH. Preferred straight or branched alkenyl groups include allyl, ethenyl, propenyl, butenyl, pentenyl, hexenyl, octenyl, nonenyl, decenyl, undecenyl, dodecenyl, tridecenyl, tetradecenyl, pentadecenyl or hexadecenyl.

The term "alkyl" includes straight or branched chain aliphatic hydrocarbon groups that are saturated and have 1 to 30 carbon atoms. The alkyl groups may be interrupted by one or more heteroatoms, such as oxygen, nitrogen, or sulfur, and may be substituted with other groups, such as, halo, -CF₃, -NO₂, -NH₂, -CN, -OCH₃, -C₆H₅, -C₆H₅O-alkyl, -O-C₆H₅O-alkenyl, p-NHC(=O)-C₆H₅-NHC(=O)-CH₃, -CH=NH, -NHC(=O)-Ph and -SH. Preferred straight or branched alkyl groups include methyl, ethyl, propyl, isopropyl, butyl, t-butyl, sec-butyl, pentyl, hexyl, heptyl, octyl, nonyl, decyl, undecyl, docecyl, tridecyl, tetradecyl, pentadecyl or hexadecyl.

The term "halo" means an element of the halogen family. Preferred halo moieties include fluorine, chlorine, bromine or iodine.

The present invention is also directed to methods of both preserving and stabilizing pharmaceutical emulsions using compounds of formula (I).

The preferred compounds of formula (I) are those wherein
R1 and R2 are independently C₁-C₆ alkyl;
R3 is C₆-C₁₆ alkyl, optionally interrupted by NHC(=O)-;
x is 2;
a is 1;
B is O⁻;
A is halo;
Y is selected from the group consisting of OH, O-C₁-C₁₀ alkyl and O-C₁-C₁₀ alkenyl; and
M is selected from the group consisting of sodium and potassium.

The more preferred compounds of formula (I) are identified as Compounds 1-3 in the following table:

| **SUBSTITUENT** | **COMPOUND 1** | **COMPOUND 2** | **COMPOUND 3** |
|---|---|---|---|
| **R1** | -CH₃ | -CH₃ | -CH₃ |
| **R2** | -CH₃ | -CH₃ | -CH₃ |
| **R3** | -(CH₂)₁₁CH₃ | -(CH₂)₃-NHC(=O)-(CH₂)₁₀CH₃ | -(CH2)₃-NHC(=O)-(CH₂)₁₂CH₃ |
| **A** | cr | Cl⁻ | Cl⁻ |
| **M** | Na⁺ | Na⁺ | Na⁺ |
| **Y** | -OH | -OH | -OH |
| **X** | 2 | 2 | 2 |
| **y** | 1 | 1 | 1 |
| **B** | -O⁻ | -O⁻ | -O⁻ |

Compound 1 is the most preferred compound of formula (I). Compound 1 is also designated as sodium coco-diimonium chloride phosphate (SCDCP) in this application.

The compounds of formula (I) can be synthesized in accordance with known procedures (see for example, U.S. Patent No. 5,286,719; 5,648,348 and 5,650,402) and/or purchased from commercial sources, such as Uniqema (Cowick Hall, Snaith Goole, East Yorkshire (UK) DN14 9AA).

The emulsions of the present invention contain one or more compounds of formula (I) in an amount sufficient to both stabilize and preserve the emulsion. In general, the amount of compound of formula (I) will be from 0.05 to 0.2%.

Embodiments of the present invention include self-stabilizing self-preserved pharmaceutical emulsions. The term self-stabilizing means that the emulsion is made up of materials which alone would not serve to provide a stable emulsion, however, due to the addition of the compounds of formula (I), it is possible to form a stable emulsion. In some embodiments, the emulsions are free of additional stabilizing agents. In other embodiments, the emulsions are substantially free of conventional stabilizing agents, i.e., while they may contain small amounts of stabilizing agents, the amounts are generally less than would be required of emulsions which do not include the compounds of formula (I), for example, less than about 2 w/v %. In still other embodiments, conventional stabilizing agents are included to improve or optimize properties of the emulsions.

The term self-preserved means that, due to the compounds of formula (I), the inclusion of conventional preservatives is typically not necessary for effective preservation of the emulsions. In some embodiments, the emulsions are free of conventional preserving agents. In other embodiments, the emulsions are substantially free of conventional preservatives, i.e., while they may contain small amounts of conventional antimicrobial agents, the amounts are generally less than would be required of emulsions which do not include the compounds of formula (I), for example, less than about 0.01 w/v %. In still other embodiments, conventional preserving agents are included to improve or optimize the preservation of the emulsions.

In this context, conventional preserving agents include, without limitation, benzalkonium chloride, benzalkonium bromide, benzethonium chloride, benzyl alcohol, phenyl ethyl alcohol, cetrimide, polyquaternium-1, chlorhexidine, chlorobutanol, cetylpyridinium chloride, parabens, thimerosal, chlorine dioxide, stabilized oxychloro compounds, PVP-Iodine complex, polyhexamethylene biguanide, alexidine, N-alkyl-2-pyrrolidone, hexetidine, sorbic acid, potassium sorbate, N,N-dichlorotaruine and mercurial preservatives.

The ophthalmic, otic and nasal emulsions of the present invention will be formulated so as to be compatible with the tissues of the eye, ear or nose. For example, as will be appreciated by those skilled in the art, ophthalmic compositions intended for direct application to the eye will usually be formulated so as to have a pH and tonicity, i.e., osmolality, that are compatible with the eye. The preferred pH for the ophthalmic emulsions of the present invention ranges from about 4.5 to about 9, more preferably from about 5 to about 8. The preferred range of osmolality for the ophthalmic emulsions of the present invention is from about 200 to about 350 milliOsmoles per kilogram (mOsm/kg).

Although polymers may not be required to stabilize the emulsions of the present invention, they may be added, for example, in artificial tear or dry eye formulations. Suitable polymers for use with the emulsions of the present invention include, but are not limited to, Carboxymethylcellulose (CMC), Guar, Hydroxypropyl Guar (HP Guar), Dextran, Xanthan and HPMC.

Osmolytes may be added. Suitable osmolytes include, but are not limited to, sorbitol, mannitol, dextran, propylene glycol and glycerin.

Corn Oil may be employed as the lipid or oil phase of the emulsion. Other oils can be used as well, for example, medium-chain triglyceride (MCT) oil, sesame oil, cottonseed oil, mineral oil or olive oil.

Boric acid may be used as a buffering agent. Other suitable buffering agents may be used in appropriate concentrations, for example, phosphates, acetate, tromethamine or citrate.

Polyoxyl-40 Hydrogenated Castor Oil (HCO-40) may be used as an emulsifier/surfactant. Other alternative emulsifiers may be used, but it may be necessary to match the hydrophilic-lipophilic balance of the emulsifier to avoid interaction with the compounds of formula (I) in order to maintain preservation. Alternative emulsifiers include poloxamines, e.g. poloxamine 1304 ("Tetronic 1304"), poloxamers (Pluronics) and glycerides.

Unless otherwise indicated, all ingredient amounts expressed in percentage terms are presented as w/v %.

The emulsions of the present invention optionally contain a therapeutically effective amount of a therapeutic or diagnostic agent. As utilized herein, the term "therapeutic agent" means a chemical or biological composition that causes a physiological effect for a therapeutic purpose. "Therapeutic agent" therefore encompasses any agent that treats or prevents a disease or pathological condition or otherwise promotes health, including, but not limited to, drug substances, antimicrobial agents, antiseptics, antibiotics, disinfectants, and antimicrobial peptides, genetic materials including any nucleic acids, nucleotides, nucleosides, proteins, etc. The term "therapeutic agent" encompasses the singular and the plural, and thus means either one therapeutic agent or more than one therapeutic agent.

The therapeutic agent (also called drug compounds or active ingredients) that can be included in the emulsions of the present invention includes, but is not limited to, ophthalmic, otic or nasal agents that can be applied either topically or internally, for example, intraocularly. Such agents include, but are not limited to: antiglaucoma agents, anti-hypertensive agents, non-steroidal antiinflammatory agents, steroidal antiinflammatory agents, antibacterial agents, antiinfective agents, antifungal agents, antiviral agents, anticataract agents, antioxidant agents, antiallergic agents, antimetabolic agents, immunosuppresive agents, and growth factor agents. In certain embodiments of the present invention, the therapeutic agent is selected from the group comprising a receptor tyrosine kinase inhibitor (RTKi), a prostaglandin and an immunosuppressant.

While the pharmaceutical emulsions of the present invention can be effectively preserved using one or more of the compounds of formula (I) without adding a conventional antimicrobial agents such as those described above, the emulsions of the present invention may also contain in addition one or more conventional preserving agent. To the emulsions of the present invention may be added, for example, a polymeric quaternary ammonium compound as described in United States Patent No. 4,407,791 (Stark). The preferred polymeric quaternary ammonium compound is polyquaternium-1. Polymeric quaternary ammonium compounds are typically utilized in an amount of from about 0.00001 to 0.01%. For the agent polyquaternium-1, an amount of about 0.001% is typically preferred.

As will be appreciated by those skilled in the art, the emulsions of the present invention may contain a wide variety of ingredients, such as tonicity agents (e.g., sodium chloride, propylene glycol, mannitol), surfactants (e.g., polysorbate, polyethoxylated castor oil (e.g. Cremophors), sorbitan fatty acid esters (e.g. Span), polyethylene glycol sorbitan fatty acid esters (e.g. Tweens) and polyoxyethylene-polyoxypropylene-polyoxyethylene copolymers), viscosity adjusting agents (e.g., hydroxypropyl methyl cellulose, other cellulose derivatives, gums and derivatives of gums), buffering agents (e.g., borates, citrates, phosphates, carbonates), comfort-enhancing agents (e.g., guar gum, xanthan gum and polyvinyl pyrrolidone), stabilizing agents (e.g., EDTA, nonyl-ethyenediaminetriacetic acid) and solublizing aids.

The following examples further illustrate various embodiments of the invention. These examples are provided to aid in the understanding of the invention and are not to be construed as limitations thereof.

### Example 1

A representative sterile compounding procedure for preparing emulsions containing compounds of Formula (I), shown in Table 1A, is described as follows:
1. Hydrate the compound of Formula (I) and HCO-40 (Polyoxyl-40 Hydrogenated Castor Oil) in purified water (50% of total batch size) and filter through a 0.2µm filtering unit.
2. Combine Boric Acid and Sorbitol in 25% purified water, stir until homogeneous and filter through a 0.2 µm filtering unit.
3. Transfer content from step 1 to a beaker that will at least hold twice as much volume as total batch weight, filter Oil into the beaker using a 0.2 µm syringe filter and stir vigorously for 1 hour.
4. Add content from step 2 to content from step 3, qs to 95% with purified water and stir vigorously for 1 hour.
7. Using 20% Tris Stock Solution (S/S), adjust the pH accordingly.
8. Qs to 100% and continue to stir vigorously for ∼20 hours.

When adding a drug or therapeutic agent, combine with Oil and sonicate until homogeneous before filtering. The concentrations provided here are based on 100% of total batch weight. This procedure, or slightly modified variants thereof, was used to prepare the emulsions described in the examples that follow.

### Example 2

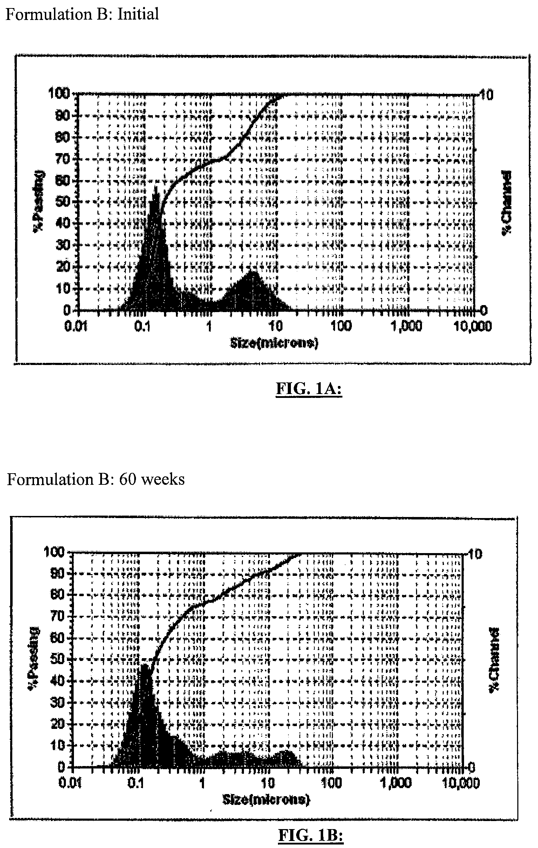

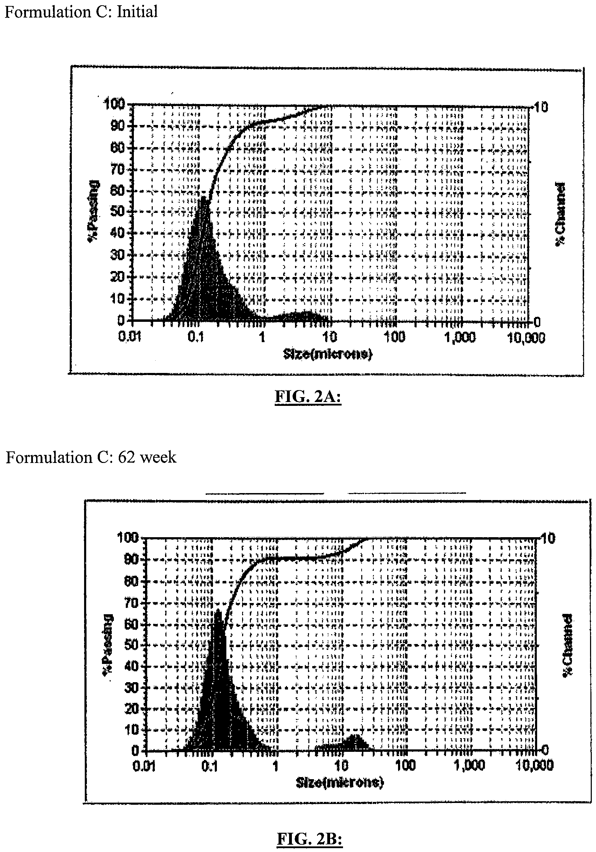

The antimicrobial activity and stability of the formulations shown in Table 1A below, which contain either 0.1 or 0.2 (w/v %) of Compound 1 were evaluated. Table 1A also contains an emulsion formulation (Formulation C) consisting of Cyclosporin and Compound 1. Table 1B constitutes overall standard preservative efficacy test (PET Screen International) results along with the results of a visual assessment of physical stability of the emulsions. The data indicates that all the formulations passed global PET standards and the emulsions are stable. Histograms for Formulation B, showing the initial particle size measurements as well as 60 weeks, can be seen in Figures 1A & 1B. Similarly, histograms for Formulation C, containing 0.05% Cyclosporin, showing the initial particle size measurements as well as at 62 weeks, can be seen in Figures 2A & 2B. It is clear from the histograms that no significant change of the emulsion particle size took place over an extended period of storage, indicating emulsion stability. The microbiological evaluation was conducted by determining the extent to which the emulsions reduced an initial population of about 10⁶ cfu/mL microorganisms over time. The abbreviation "cfu" means colony forming unit. In the PET Screen International, at 24 hours all bacteria including S. *aureus, P. aeruginosa* and *E. coli* showed 5 log reductions for 0.1% Compound 1 and 0.2% Compound 1. Similarly, at 7 days approximately 5 log reductions were observed for *C. albicans.* Overall EPA requirement of preservation in all tested formulations was achieved. The microbiology results are provided in greater detail in Table 1C, where the results of the evaluation of five microorganisms are provided.

**TABLE 1A**

| **FORMULATION COMPOSITIONS** | | | | |
|---|---|---|---|---|
| **FORMULATION** | **A** | **B** | **C** | **D** |
| **INGREDIENT** | Concentration (w/v %) | | | |
| Compound 1 | 0.2 | 0.1 | 0.1 | 0.1 |
| Sorbitol | 0.33 | 0.33 | 0.33 | 0.33 |
| Boric Acid | 1 | 1 | 1 | 1 |
| HCO-40 | 0.5 | 0.5 | 0.5 | 0.5 |
| Corn Oil | 0.75 | 0.75 | 0.75 | 0.75 |
| Cyclosporine | None | None | 0.05 | None |
| 20% Tris S/S* | Adjust pH 7.2 | Adjust pH 7.2 | Adjust pH 7.2 | Adjust pH 6.0 |
| Purified Water | Qs to 100% | Qs to 100% | Qs to 100% | Qs to 100% |

| | | | | |
|---|---|---|---|---|
| *S/S=Stock Solution | | | | |

**TABLE 1B**

| **PHYSICAL AND MICROBIOLOGICAL PARAMETERS** | | | | |
|---|---|---|---|---|
| **FORMULATION** | **A** | **B** | **C** | **D** |
| Final pH | 7.2 | 7.2 | 7.2 | 6.0 |
| PET Screen | Pass EPA^{a} | Pass EPA^{a} | Pass EPA^{a} | Pass EPA^{a} |
| Visual Stability | No visible separation after 11 days at 40°C and 50°C. No visible separation at RT for 60 weeks. | Minimal separation after 11 days at 40°C and 50°C, cleared upon inverting once. No visible separation at RT for 60 weeks. | No visible separation after 63 weeks at RT. | No visible separation after 9 weeks atRT. |

| | | | | |
|---|---|---|---|---|
| ^{a}Projected to pass EPA based on PET screen results | | | | |

**TABLE 1C**

| **MICROBIOLOGY RESULTS** | | | | |
|---|---|---|---|---|
| **Formulation** | **A** | **B** | **C** | **D** |
| Time/Test Organism | Log Order Reduction | | | |
| 6 hour / S.aureus | N/A | N/A | 4.9 | 4.9 |
| 24 hour/ S. aureus | 5.0 | 5.0 | 4.9 | 4.9 |
| 7 day/ S. aureus | 5.0 | 5.0 | 4.9 | 4.9 |
| 6 hour/ P. aeruginosa | N/A | N/A | 5.0 | 5.0 |
| 24 hour/ P. aeruginosa | 5.0 | 5.0 | 5.0 | 5.0 |
| 7 day / P. aeruginosa | 5.0 | 5.0 | 5.0 | 5.0 |
| 6 hour / E. coli | N/A | N/A | 4.9 | 3.1 |
| 24 hour / E. coli | 5.0 | 5.0 | 4.9 | 5.0 |
| 7 day / E. coli | 5.0 | 5.0 | 4.9 | 5.0 |
| 7 day / C. albicans | 5.0 | 5.0 | 5.0 | 4.9 |
| 7 day/ A. niger | 3.9 | 3.9 | 2.8 | 2.6 |

| | | | | |
|---|---|---|---|---|
| *N/A= Not applicable | | | | |

### Example 3

The stability and antimicrobial activity of the formulations shown in Table 2A below, which contain either 0.1, 0.2 (w/v %) of Compound 1, or have no added compound of Formula (I), were evaluated. The results, shown in Table 2B below, demonstrate the stabilizing effect of adding the compound of Formula (I) to the emulsions.

**TABLE 2A**

| **FORMULATION COMPOSITIONS** | | | |
|---|---|---|---|
| **FORMULATION** | **A** | **B** | **E** |
| **INGREDIENTS** | Concentration (w/v %) | | |
| Compound 1 | 0.2 | 0.1 | None |
| Sorbitol | 0.33 | 0.33 | 0.33 |
| Boric Acid | 1 | 1 | 1 |
| HCO-40 | 0.5 | 0.5 | 0.5 |
| Corn Oil | 0.75 | 0.75 | 0.75 |
| 20% Tris S/S* | Adjust pH 7.2 | Adjust pH 7.2 | Adjust pH 7.2 |
| Purified Water | Qs to 100% | Qs to 100% | Qs to 100% |

| | | | |
|---|---|---|---|
| *S/S=Stock Solution | | | |

**TABLE 2B**

| **PHYSICAL PARAMETERS** | | | |
|---|---|---|---|
| **FORMULATION** | **A** | **B** | **E** |
| Final pH | 7.2 | 7.2 | 7.2 |
| PET Screen | PASS EPA^{a} | PASS EPA^{a} | N/A |
| Visual Stability | No visible separation after 11 days at 40°C and 50°C. No visible separation at RT for 60 weeks. | Minimal separation after 11 days at 40°C and 50°C, cleared upon inverting once. No visible separation at RT for 60 weeks | Very milky and creamy. Upon allowing to sit overnight there was significant separation. |

| | | | |
|---|---|---|---|
| ^{a} Projected to pass EPA | | | |

### Example 4

Stability measurements of the formulation with higher amount of corn oil (1%) shown in Table 3A are provided in Table 3B below.

**TABLE 3A**

| **EMULSION FORMULATIONS WITH 1**% **CORN OIL** | |
|---|---|
| **FORMULATION** | **F** |
| **INGREDIENTS** | Concentration (w/v %) |
| Compound 1 | 0.1 |
| Sorbitol | 0.33 |
| Boric Acid | 1 |
| HCO-40 | 0.5 |
| Corn Oil | 1 |
| 20% Tris S/S* | Adjust pH |
| Purified Water | Qs to 100% |
| Final pH | 7.2 |

| | |
|---|---|
| *S/S=Stock Solution | |

**TABLE 3B**

| **PHYSICAL PARAMETERS** | |
|---|---|
| **FORMULATION** | **F** |
| Visual Stability | Particle size histogram showed multiple peaks at ∼0.1, 0.4 & 2 µm. No visible separation in samples stored at RT for 55 weeks. |

### Example 5

Further stability test results for Formulation C containing 0.05% Cyclosporine as active ingredient are shown in Table 4 below. The emulsion was packaged in both low density polyethylene (LDPE) Droptainers and glass vials. LDPE containers were used to measure the weight loss, pH and osmolality. The glass vials were used to examine color and physical stability of the formulations. No significant change of pH and osmolality were noted in the formulation stored at RT and 40°C. However, samples stored at 60°C showed some change of pH and osmolality. Visual assessment of emulsion at room temperature (RT) showed either a slight separation, which cleared upon inverting once, or no separation at 2, 4, 6 and 62 weeks (2w, 4w, 6w and 62w). No separation was discerned in samples stored at 40°C and 60°C for 2, 4 and 6 weeks. Emulsions stored at RT and 40°C showed no change of color whereas formulations stored at 60°C turned yellow.

**TABLE 4**

| **STABILITY RESULTS** | | | | | | |
|---|---|---|---|---|---|---|
| **Condition/Time** | | **Total Weight Loss (%)** | **pH** | **Osmolality (mOsm)** | **Color** | **Visual Stability** |
| **Packaging** | | **LDPE** | **LDPE** | **LDPE** | **Glass Vial** | **Glass Vial** |
| **Initial** | | N/A* | 7.2 | 174 | Opalescent | Stable, No Separation |
| **RT** | 2w | 0.04 | 7.1 | 172 | Opalescent | Slight separation, cleared upon inverting once. |
| | 4w | 0.05 | 7.07 | 170 | Opalescent | Slight separation, cleared upon inverting once. |
| | 6w | 0.05 | 7.09 | 170 | Opalescent | Stable, No Separation |
| | 62w | N/A | 7.07 | 179 | Opalescent | Stable, No Separation |
| **40°C** | 2w | 0.17 | 7.07 | 171 | Opalescent | Stable, No Separation |
| | 4w | 0.36 | 7.07 | 171 | Opalescent | Stable, No Separation |
| | 6w | 0.41 | 7.08 | 171 | Opalescent | Stable, No Separation |
| **60°C** | 2w | 0.84 | 6.93 | 179 | Light Yellow | Stable, No Separation |
| | 4w | 1.69 | 6.82 | 187 | Yellow | Stable, No Separation |
| | 6w | 2.33 | 6.77 | 191 | Yellow | Stable, No Separation |

| | | | | | | |
|---|---|---|---|---|---|---|
| *N/A = Not applicable | | | | | | |

### Example 6

A one-day exaggerated topical ocular irritation and comfort evaluation of three formulations was conducted. Three New Zealand White rabbits were assigned to each test group and one eye of each animal was selected for use. The formulations were administered in the test eye approximately every 30 minutes for a total of 10 doses. Immediately following the first and last dose, a comfort evaluation was performed. One hour after the last dose, the test eye was examined biomicroscopically. Twenty-four hours following the first dose, all animals were reexamined for general health.

The formulations tested were as follows:
1. 0.2 % Compound 1 Emulsion (Formulation A whose composition is as shown in Table 1A above).
2. 0.1 % Compound 1 Emulsion (Formulation B whose composition is as shown in Table 1A above).
3. 0.1 % Compound 1 Emulsion with 0.05% Cyclosporine (Formulation C whose composition is as shown in Table 1A above).

The maximum possible scores are as indicated as follows:

| | |
|---|---|
| Conjunctival Congestion (Conj. Cong.) | 3.0 |
| Conjunctival Swelling (Conj. Swell.) | 4.0 |
| Conjunctival Discharge (Conj. Disch.) | 3.0 |
| Iritis | 4.0 |
| Flare | 3.0 |
| Light Reflex | 2.0 |
| Corneal Cloudiness | 4.0 |
| Corneal Area | 4.0 |
| Fluorescein Intensity (Fluor. Inten.) | 4.0 |
| Fluorescein Area (Fluor. Area) | 4.0 |
| Comfort (CMFT) | 4.0 |

The results of these studies, presented in Table 5A, 5B and 5C, in particular considering the exaggerating dosage regimen employed, indicate that these formulations are substantially comfortable and not adversely irritating to the eye upon topical ocular administration.

**TABLE 5A**

| **One-Day Topical Ocular Irritation and Comfort Evaluation Formulation A** | | | | | | | | | | | | | | |
|---|---|---|---|---|---|---|---|---|---|---|---|---|---|---|
| | **Conj. Cong.** | **Conj. Swell.** | **Conj. Disch.** | **Total Conj. Irrit.** | **Light Reflex** | **Flare** | **Iritis** | **Corneal Cloudy** | **Corneal Area** | **Fluor. Inten.** | **Fluor. Area** | **1^{st} CMFT** | **2^{nd} CMFT** | **Total Comfort** |
| MEAN | 0.7 | 0.0 | 0.0 | - | 0.3 | 0.0 | 0.0 | 0.0 | 0.0 | 0.0 | 0.0 | 2.0 | 2.0 | - |
| INCIDENCE | 2/3 | 0/3 | 0/3 | - | 1/3 | 0/3 | 0/3 | 0/3 | 0/3 | 0/3 | 0/3 | 3/3 | 3/3 | - |
| SEVERITY | N/A | N/A | N/A | Minimal | Minimal | None | None | None | None | None | None | N/A | N/A | Moderate |

**TABLE 5B**

| **One-Day Topical Ocular Irritation and Comfort Evaluation Formulation B** | | | | | | | | | | | | | | |
|---|---|---|---|---|---|---|---|---|---|---|---|---|---|---|
| | **Conj. Cong.** | **Conj. Swell.** | **Conj. Disch.** | **Total Conj. Irrit.** | **Light Reflex** | **Flare** | **Iritis** | **Corneal Cloudy** | **Corneal Area** | **Fluor. Inten.** | **Fluor. Area** | **1^{st} CMFT** | **2^{nd} CMFT** | **Total Comfort** |
| MEAN | 0.3 | 0.0 | 0.0 | - | 0.0 | 0.0 | 0.0 | 0.0 | 0.0 | 0.0 | 0.0 | 1.7 | 1.3 | - |
| INCIDENCE | 1/3 | 0/3 | 0/3 | - | 0/3 | 0/3 | 0/3 | 0/3 | 0/3 | 0/3 | 0/3 | 3/3 | 3/3 | - |
| SEVERITY | N/A | N/A | N/A | Minimal | None | None | None | None | None | None | None | N/A | N/A | Minimal - Moderate |

**TABLE 5C**

| **One-Day Topical Ocular Irritation and Comfort Evaluation Formulation C** | | | | | | | | | | | | | | |
|---|---|---|---|---|---|---|---|---|---|---|---|---|---|---|
| | **Conj. Cong.** | **Conj. Swell.** | **Conj. Disch.** | **Total Conj. Irrit.** | **Light Reflex** | **Flare** | **Iritis** | **Corneal Cloudy** | **Corneal Area** | **Fluor. Inten.** | **Fluor. Area** | **1^{st} CMFT** | **2^{nd} CMFT** | **Total Comfort** |
| MEAN | 0.7 | 0.0 | 0.0 | - | 0.0 | 0.0 | 0.0 | 0.0 | 0.0 | 0.0 | 0.0 | 1.7 | 1.3 | - |
| INCIDENCE | 1/3 | 0/3 | 0/3 | - | 0/3 | 0/3 | 0/3 | 0/3 | 0/3 | 0/3 | 0/3 | 3/3 | 3/3 | - |
| SEVERITY | N/A | N/A | N/A | Minimal | None | None | None | None | None | None | None | N/A | N/A | Minimal-Moderate |

### Example 7

**Self-Preserved Emulsion to Treat Dry Eye**

| **Formulation** | **G** |
|---|---|
| Components | Amount (%w/v) |
| Cyclosporine | 0.05 |
| Polyoxyl 40 Hydrogenated Castor Oil | 0.75 |
| Compound No. 1 | 0.1 |
| MCT Oil | 2 |
| Boric acid | 1 |
| Sorbitol | 0.33 |
| Propylene glycol | 0.7 |
| Hydrochloric acid/ Sodium hydroxide | q.s. to pH |
| Purified water | q.s. to 100% |
| pH | 6.0 |

**Self Preserved Emulsion to Treat Glaucoma**

| **Formulation** | **H** |
|---|---|
| Components | Amount (%w/v) |
| Travoprost | 0.004 |
| Polyoxyl 40 Hydrogenated Castor Oil | 0.75 |
| Compound No. 1 | 0.1 |
| MCT Oil | 2 |
| Boric acid | 1 |
| Sorbitol | 0.33 |
| Propylene glycol | 0.7 |
| Mannitol | 0.5 |
| Hydrochloric acid/ Sodium hydroxide | q.s. to pH |
| Purified water | q.s. to 100% |
| pH | 6.0 |

**Self Preserved Emulsion to Treat Glaucoma**

| **Formulation** | **I** |
|---|---|
| Components | Amount (%w/v) |
| Latanoprost | 0.005 |
| Polyoxyl 40 Hydrogenated Castor Oil | 0.75 |
| Compound No. 1 | 0.1 |
| MCT Oil | 2 |
| Boric acid | 1 |
| Sorbitol | 0.33 |
| Propylene glycol | 0.7 |
| Mannitol | 0.5 |
| Hydrochloric acid/ Sodium hydroxide | q.s. to pH |
| Purified water | q.s. to 100% |
| pH | 6.0 |

**Preservative Efficacy Data of the Emulsions**

| **Formulation** | **I** | **H** | **G** |
|---|---|---|---|
| PET | Log ₁₀Unit Reduction | | |
| S. aureus/ 6 h | 5.0 | 5.0 | 5.0 |
| S. aureus/ 24 h | 5.0 | 5.0 | 5.0 |
| S. aureus/ 7 d | 5.0 | 5.0 | 5.0 |
| S. aureus/14 d | 5.0 | 5.0 | 5.0 |
| S. aureus/ 28 d | 5.0 | 5.0 | 5.0 |
| P. aerugin/6 h | 4.9 | 4.9 | 4.9 |
| P. aerugin/24 h | 4.9 | 4.9 | 4.9 |
| P. aerugin/7 d | 4.9 | 4.9 | 4.9 |
| P. aerugin/14 d | 4.9 | 4.9 | 4.9 |
| P. aerugin/28 d | 4.9 | 4.9 | 4.9 |
| E. coli/6 h | 5.0 | 5.0 | 5.0 |
| E. coli/24h | 5.0 | 5.0 | 5.0 |
| E. coli/7d | 5.0 | 5.0 | 5.0 |
| E. coli/14d | 5.0 | 5.0 | 5.0 |
| E. coli/28d | 5.0 | 5.0 | 5.0 |
| C. albican/7 d | 4.9 | 4.9 | 4.9 |
| C. albican/14 d | 4.9 | 4.9 | 4.9 |
| C. albican/28 d | 4.9 | 4.9 | 4.9 |
| A. niger/7 d | 3.7 | 4.5 | 4.6 |
| A. niger/14 d | 5.1 | 5.1 | 5.1 |
| A. niger/28 d | 5.1 | 5.1 | 5.1 |
| RESULTS | EPA | EPA | EPA |

**One-day Acute Ocular Safety / Comfort Study Data of Self-Preserved Emulsions**

| **Formulation** | **Description** | **Conj. Irritation** | **Comfort Score** | **Pass/Fail** |
|---|---|---|---|---|
| G* | 0.05% Cyclosporine Self Preserved Emulsion | 2.0 (Minimal) | 4.0 (Moderate) | Pass |
| I | 0.005% Latanaprost Self Preserved Emulsion | 2.0 (Minimal) | 3.7 (Moderate) | Pass |
| H | 0.004% Travoprost Self Preserved Emulsion | 1.8 (Minimal) | 3.3 (Moderate) | Pass |

### Example 8

### A Representative Preserved Formulation

*concentrations for Compound of Formula (I) outside of 0.05 to 0.2% are not within the scope of the claims.

| Ingredient | Amount (w/w %) |
|---|---|
| Active | 0 - 2.0% |
| Compound of Formula (I) | 0.001-1.0% |
| Sorbitol | 0.33% |
| Boric Acid | 1.0% |
| Corn Oil | 0.1-5.0% |
| HCO-40 | 0.1-5.0% |
| 20% Tromethamine S/S | q.s. to pH 7.2 +/- 0.2 |
| Purified Water | q.s. 100% |

### Example 9

### A Representative Preserved Formulation

*concentrations for Compound of Formula (I) outside of 0.05 to 0.2% are not within the scope of the claims.

| Ingredient | Amount (w/w %) |
|---|---|
| Active | 0 - 2.0% |
| Compound of Formula (I) | 0.001-1.0% |
| Sorbitol | 0.33% |
| Boric Acid | 1.0% |
| MCT Oil | 0.1-5.0% |
| Tetronic 1304 | 0.1-5.0% |
| 20% Tromethamine S/S | q.s. to pH 7.2 +/- 0.2 |
| Purified Water | q.s. 100% |

### Example 10

### A Representative Preserved Formulation for Dry Eye Care

*concentrations for Compound of Formula (I) outside of 0.05 to 0.2% are not within the scope of the claims.

| Ingredient | Amount (w/w %) |
|---|---|
| Active | 0 - 2.0% |
| Compound of Formula (I) | 0.001-1.0% |
| Guar Guma^{a} | 0.16 |
| Sorbitol | 0.33% |
| Boric Acid | 1.0% |
| MCT Oil | 0.1-5.0% |
| HCO 40^{b} | 0.1-5.0% |
| 20% Tromethamine S/S | q.s. to pH 7.2 +/- 0.2 |
| Purified Water | q.s. 100% |

| | |
|---|---|
| ^{a} could be replaced with hydroxypropyl guar gum ^{b} other suitable surfactants such as Tween, Span, Cremophore etc. may also be used either alone or in combination | |

The invention in its broader aspects is not limited to the specific details shown and described above. Departures may be made from such details within the scope of the accompanying claims without departing from the principles of the invention and without sacrificing its advantages.

## Claims

1. An emulsion suitable for ocular, nasal or otic administration comprising water,
a buffering agent,
an oil,
an emulsifier,
a pH adjuster,
a multifunctional synthetic compound of formula I, 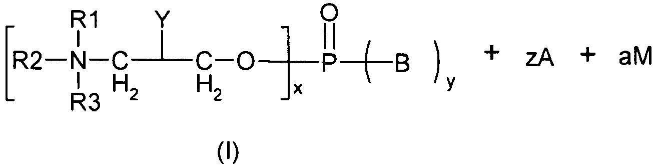
wherein:
x is 1 to 3 or mixtures thereof;
x+y is equal to 3;
a is 0 to 2;
z is equal to x;
B is O- or OM;
A is an anion;
M is a cation;
Y is selected from the group consisting of OH, O-C₁-C₁₀ alkyl and O-C₁-C₁₀ alkenyl;
and
R1, R2 and R3 are the same or different and are alkyl, substituted alkyl, alkyl aryl or alkenyl groups, optionally interrupted by -NHC(=O)-, of up to 16 carbon atoms with the proviso that the total carbon atoms in R1+R2+R3 is between 10 and 24,
and optionally a therapeutic agent, wherein the amount of the compound of formula I is from 0.05 to 0.2%.

2. An emulsion according to claim 1, wherein:
R1 and R2 are independently C₁-C₆ alkyl;
R3 is C₆-C₁₆ alkyl, optionally interrupted by -NHC(=O)-;
x is 2;
a is 1;
B is O-;
A is halo;
Y is selected from the group consisting of OH, O-C₁-C₁₀ alkyl and 0-C₁-C₁₀ alkenyl; and
M is selected from the group consisting of sodium and potassium.

3. An emulsion according to claim 2, wherein:
R1 is methyl;
R2 is methyl;
R3 is -(CH2)₁₁CH₃;
A is Cl⁻;
Y is OH;
and
M is Na⁺

4. An emulsion according to claim 1, wherein the emulsion is free of a biocidal agent other than a compound of Formula I.

5. An emulsion according to claim 1, wherein the emulsion is substantially free of a biocidal agent other than a compound of Formula I.

6. An emulsion according to claim 1, wherein the emulsion does not require heating, homogenization or thickening agents for emulsion stabilization.

7. An emulsion according to claim 1, wherein the emulsion further comprises a demulcent selected from the group consisting of HPMC, HEC, CMC, Guar gum and Xanthan gum.

8. An emulsion according to claim 1, wherein the optional therapeutic agent, when present, is selected from the group consisting of ophthalmic, otic and nasal agents.

9. An emulsion according to claim 1, wherein the emulsion is non-irritating to ocular tissues.

10. A method of both preserving and stabilizing an emulsion for pharmaceutical use, comprising adding to the emulsion a stabilizing and preserving amount of a multifunctional compound of formula (I), 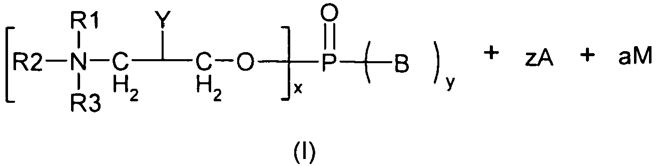 wherein:
x is 1 to 3 or mixtures thereof;
x+y is equal to 3;
a is 0 to 2;
z is equal to x;
B is O- or OM;
A is an anion;
M is a cation;
Y is selected from the group consisting of OH, O-C₁-C₁₀ alkyl and O-C₁-C₁₀ alkenyl;
and
R1, R2 and R3 are the same or different and are alkyl, substituted alkyl, alkyl aryl or alkenyl groups, optionally interrupted by -NHC(=O)-, of up to 16 carbon atoms with the proviso that the total carbon atoms in R1+R2+R3 is between 10 and 24, wherein the amount of the compound of formula I is from 0.05 to 0.2%.

11. A method according to claim 10, wherein:
R1 and R2 are independently C₁-C₆ alkyl;
R3 is C₆-C₁₆ alkyl, optionally interrupted by -NHC(=O)-;
x is 2;
a is 1;
B is O⁻;
A is halo;
Y is selected from the group consisting of OH, O-C₁-C₁₀ alkyl and O-C₁-C₁₀ alkenyl; and
M is selected from the group consisting of sodium and potassium.

12. A method according to claim 11, wherein:
R1 is methyl;
R2 is methyl;
R3 is -(CH2)₁₁CH₃;
A is Cl⁻;
Y is OH;
and
M is Na⁺

13. A method according to claim 10, wherein the emulsion further comprises a therapeutic agent.

14. A method according to claim 10, wherein the emulsion further comprises a demulcent selected from the group consisting of HPMC, HEC, CMC, Guar gum and Xanthan gum.

15. A method of preparing an emulsion suitable for ophthalmic, otic or nasal administration, wherein to a composition comprising a buffering agent, an oil, an emulsifier, a pH adjuster and optionally a therapeutic agent is added, in an amount sufficient to both preserve and stabilize said composition, a multifunctional synthetic compound of formula (I) 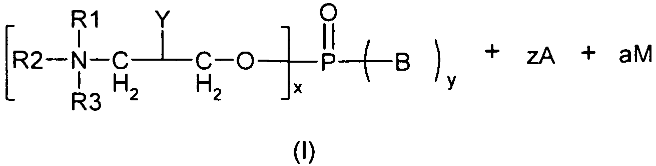 wherein:
x is 1 to 3 or mixtures thereof;
x+y is equal to 3;
a is 0 to 2;
z is equal to x;
B is O- or OM;
A is an anion;
M is a cation;
Y is selected from the group consisting of OH, O-C₁-C₁₀ alkyl and O-C₁-C₁₀ alkenyl;
and
R1, R2 and R3 are the same or different and are alkyl, substituted alkyl, alkyl aryl or alkenyl groups, optionally interrupted by -NHC(=O)-, of up to 16 carbon atoms with the proviso that the total carbon atoms in R1+R2+R3 is between 10 and 24, wherein the amount of the compound of formula I is from 0.05 to 0.2%.

16. A method according to claim 15, wherein:
R1 and R2 are methyl
R3 is selected from the group consisting of (CH₂)₁₁CH₃, (CH₂)₃-NHC(=O)-(CH₂)₁₀CH₃, and -(CH₂)₃-NHC(=O)-(CH₂)₁₂CH₃;
x is 2;
a is 1;
B is O-;
A is chloro;
Y is OH; and
M is an alkali metal ion.

17. A method according to claim 16, wherein:
R1 is methyl;
R2 is methyl;
R3 is -(CH₂)₁₁CH₃;
Y is OH;
and
M is Na⁺

18. A method according to claim 15, wherein the emulsion further comprises a demulcent selected from the group consisting of HPMC, HEC, CMC, Guar gum and Xanthan gum.

19. A method according to claim 15, wherein the optional therapeutic agent, when present, is selected from the group consisting of ophthalmic, otic and nasal agents.

## Patentansprüche

1. Emulsion, geeignet zur Verabreichung ins Auge, die Nase oder das Ohr, umfassend Wasser,
einen Puffer,
ein Öl,
einen Emulgator,
ein pH-Einstellmittel,
eine multifunktionale synthetische Verbindung der Formel I, 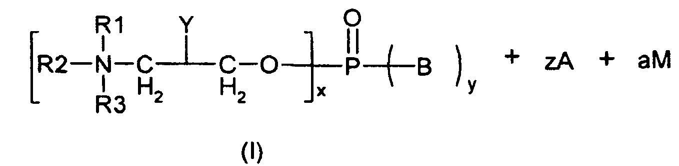
wobei:
x 1 bis 3 oder Gemische davon ist;
x + y gleich 3 ist;
a 0 bis 2 ist;
z gleich x ist;
B O- oder OM ist;
A ein Anion ist;
M ein Kation ist;
Y ausgewählt ist aus der Gruppe, bestehend aus OH, O-C₁-C₁₀-Alkyl und O-C₁-C₁₀-Alkenyl;
und
R1, R2 und R3 gleich oder verschieden sind und Alkyl-, substituierte Alkyl-, Alkylaryl-oder Alkenylgruppen, gegebenenfalls unterbrochen durch -NHC(=O)-, von bis zu 16 Kohlenstoffatomen sind, mit der Maßgabe, dass die Kohlenstoffatome in R1+R2+R3 insgesamt zwischen 10 und 24 betragen,
und gegebenenfalls ein Therapeutikum, wobei die Menge der Verbindung der Formel I 0,05 bis 0,2 % beträgt.

2. Emulsion nach Anspruch 1, wobei:
R1 und R2 unabhängig C₁-C₆-Alkyl sind;
R3 C₆-C₁₆-Alkyl, gegebenenfalls unterbrochen durch -NHC(=O)-, ist;
x 2 ist;
a 1 ist;
B O⁻ ist;
A Halogen ist;
Y ausgewählt ist aus der Gruppe, bestehend aus OH, O-C₁-C₁₀-Alkyl und O-C₁-C₁₀-Alkenyl; und
M ausgewählt ist aus der Gruppe, bestehend aus Natrium und Kalium.

3. Emulsion nach Anspruch 2, wobei:
R1 Methyl ist;
R2 Methyl ist;
R3 -(CH₂)₁₁CH₃ ist;
A Cl⁻ ist;
Y OH ist
und
M Na⁺ ist.

4. Emulsion nach Anspruch 1, wobei die Emulsion frei von einem anderen Biozid als der Verbindung der Formel I ist.

5. Emulsion nach Anspruch 1, wobei die Emulsion im Wesentlichen frei von einem anderen Biozid als der Verbindung der Formel I ist.

6. Emulsion nach Anspruch 1, wobei die Emulsion zur Emulsionsstabilisierung keiner Erhitzung, Homogenisierung oder Verdickungsmittel bedarf.

7. Emulsion nach Anspruch 1, wobei die Emulsion ferner ein Linderungsmittel, ausgewählt aus der Gruppe, bestehend aus HPMC, HEC, CMC, Guargummi und Xanthan-Gummi, umfasst.

8. Emulsion nach Anspruch 1, wobei das optionale Therapeutikum, sofern vorhanden, ausgewählt ist aus der Gruppe, bestehend aus Augen-, Ohren- und Nasenmitteln.

9. Emulsion nach Anspruch 1, wobei die Emulsion okulare Gewebe nicht reizt.

10. Verfahren zur Konservierung und Stabilisierung einer Emulsion zur pharmazeutischen Verwendung, umfassend die Zugabe einer stabilisierenden und konservierenden Menge einer multifunktionalen Verbindung der Formel (I), 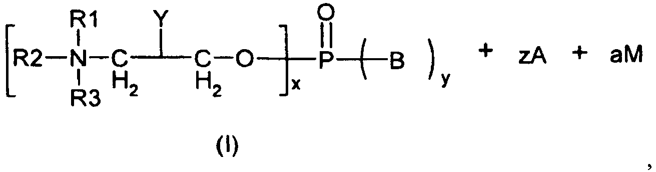 wobei:
x 1 bis 3 oder Gemische davon ist;
x + y gleich 3 ist;
a 0 bis 2 ist;
z gleich x ist;
B O- oder OM ist;
A ein Anion ist;
M ein Kation ist;
Y ausgewählt ist aus der Gruppe, bestehend aus OH, O-C₁-C₁₀-Alkyl und O-C₁-C₁₀-Alkenyl;
und
R1, R2 und R3 gleich oder verschieden sind und Alkyl-, substituierte Alkyl-, Alkylaryl-oder Alkenylgruppen, gegebenenfalls unterbrochen durch -NHC(=O)-, von bis zu 16 Kohlenstoffatomen sind, mit der Maßgabe, dass die Kohlenstoffatome in R1+R2+R3 insgesamt zwischen 10 und 24 betragen, wobei die Menge der Verbindung der Formel I 0,05 bis 0,2 % beträgt,
zu der Emulsion.

11. Verfahren nach Anspruch 10, wobei:
R1 und R2 unabhängig C₁-C₆-Alkyl sind;
R3 C₆-C₁₆-Alkyl, gegebenenfalls unterbrochen durch -NHC(=O)-, ist;
x 2 ist;
a 1 ist;
B O⁻ ist;
A Halogen ist;
Y ausgewählt ist aus der Gruppe, bestehend aus OH, O-C₁-C₁₀-Alkyl und O-C₁-C₁₀-Alkenyl;
und
M ausgewählt ist aus der Gruppe, bestehend aus Natrium und Kalium.

12. Verfahren nach Anspruch 11, wobei:
R1 Methyl ist;
R2 Methyl ist;
R3 -(CH_{Z})₁₁CH₃ ist;
A Cl⁻ ist;
Y OH ist
und
M Na⁺ ist.

13. Verfahren nach Anspruch 10, wobei die Emulsion ferner ein Therapeutikum umfasst.

14. Verfahren nach Anspruch 10, wobei die Emulsion ferner ein Linderungsmittel, ausgewählt aus der Gruppe, bestehend aus HPMC, HEC, CMC, Guargummi und Xanthan-Gummi, umfasst.

15. Verfahren zur Herstellung einer Emulsion, geeignet zur Verabreichung ins Auge, das Ohr oder die Nase, wobei einer Zusammensetzung, umfassend einen Puffer, ein Öl, einen Emulgator, ein pH-Einstellmittel und gegebenenfalls ein Therapeutikum, in einer Menge, die ausreicht, die Zusammensetzung zu konservieren und zu stabilisieren, eine multifunktionale, synthetische Verbindung der Formel (I) 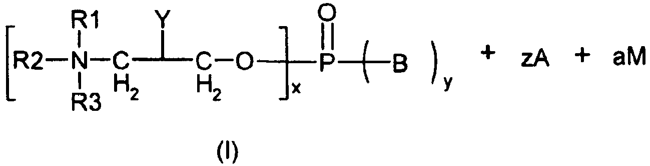 wobei:
x 1 bis 3 oder Gemische davon ist;
x + y gleich 3 ist;
a 0 bis 2 ist;
z gleich x ist;
B O- oder OM ist;
A ein Anion ist;
M ein Kation ist;
Y ausgewählt ist aus der Gruppe, bestehend aus OH, O-C₁-C₁₀-Alkyl und O-C₁-C₁₀-Alkenyl;
und
R1, R2 und R3 gleich oder verschieden sind und Alkyl-, substituierte Alkyl-, Alkylaryl-oder Alkenylgruppen, gegebenenfalls unterbrochen durch -NHC(=O)-, von bis zu 16 Kohlenstoffatomen sind, mit der Maßgabe, dass die Kohlenstoffatome in R1+R2+R3 insgesamt zwischen 10 und 24 betragen, wobei die Menge der Verbindung der Formel I 0,05 bis 0,2 % beträgt,
zugegeben wird.

16. Verfahren nach Anspruch 15, wobei:
R1 und R2 Methyl sind;
R3 ausgewählt ist aus der Gruppe, bestehend aus (CH₂)₁₁CH₃, (CH₂)₃-NHC(=O)-(CH₂)₁₀CH₃ und -(CH₂)₃-NHC(=O)-(CH₂)₁₂CH₃;
x 2 ist;
a 1 ist;
B O- ist;
A Chlor ist;
Y OH ist und
M ein Alkali(metall)ion ist.

17. Verfahren nach Anspruch 16, wobei:
R1 Methyl ist;
R2 Methyl ist;
R3 -(CH₂)₁₁CH₃ ist;
Y OH ist
und
M Na⁺ ist.

18. Verfahren nach Anspruch 15, wobei die Emulsion ferner ein Linderungsmittel, ausgewählt aus der Gruppe, bestehend aus HPMC, HEC, CMC, Guargummi und Xanthan-Gummi, umfasst.

19. Verfahren nach Anspruch 15, wobei das optionale Therapeutikum, sofern vorhanden, ausgewählt ist aus der Gruppe, bestehend aus Augen-, Ohren- und Nasenmitteln.

## Revendications

1. Émulsion adaptée pour administration oculaire, nasale ou otique comprenant
de l'eau,
un agent tampon,
une huile,
un émulsifiant,
un ajusteur de pH,
un composé synthétique plurifonctionnel de formule I, 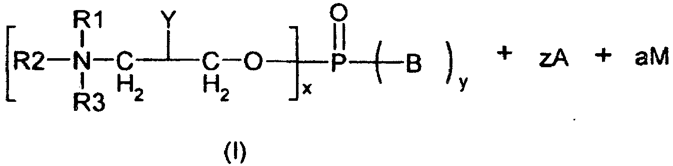
dans laquelle
x est égal à 1 à 3 ou des mélanges correspondants ;
x+y est égal à 3 ;
a est égal à 0 à 2 ;
z est égal à x ;
B est O- ou OM ;
A est un anion,
M est un cation,
Y est choisi dans le groupe constitué d'OH, O-C₁-C₁₀ alkyle et O- C₁-C₁₀-alcényle,
et
R1, R2 et R3 sont identiques ou différents et sont des groupes alkyle, alkyle substitués, alkyl aryle ou alcényle, éventuellement interrompus par un -NHC(=O)-, pouvant atteindre 16 atomes de carbone, à condition que le total des atomes de carbone dans R1+R2+R3 soit compris entre 10 et 24, et éventuellement un agent thérapeutique, dans lequel la quantité du composé de formule I est de 0,05 à 0,2%.

2. Émulsion selon la revendication 1, dans laquelle :
R1 et R2 sont indépendamment un C₁-C₆ alkyle ;
R3 est un C₆-C₁₆ alkyle, éventuellement interrompu par -NHC(=O)-;
x est égal à 2 ;
a est égal à 1 ;
B est O-;
A est un halo ;
Y est choisi dans le groupe constitué d'OH, O-C₁-C₁₀ alkyle et O-C₁-C₁₀ alcényle ; et
M est choisi dans le groupe constitué de sodium et de potassium.

3. Émulsion selon la revendication 2, dans laquelle :
R1 est un méthyle ;
R2 est un méthyle ;
R3 est -(CH2)₁₁CH₃;
A est Cl⁻;
YestOH;
et
M est Na⁺.

4. Émulsion selon la revendication 1, dans laquelle l'émulsion est exempt d'agent biocide autre qu'un composé de Formule I.

5. Émulsion selon la revendication 1, dans laquelle l'émulsion est pratiquement exempt d'agent biocide autre qu'un composé de Formule I.

6. Émulsion selon la revendication 1, dans laquelle l'émulsion ne nécessite pas de chauffage, d'homogénéisation ou d'agents épaississants pour la stabilisation de l'émulsion.

7. Émulsion selon la revendication 1, dans laquelle l'émulsion comprend en outre un agent émollient choisi dans le groupe constitué d'HPMC, HEC, CMC, gomme de Guar et gomme Xanthane.

8. Émulsion selon la revendication 1, dans laquelle l'agent thérapeutique optionnel, lorsqu'il est présent, est choisi dans le groupe constitué d'agents ophtalmiques, otiques et nasaux.

9. Émulsion selon la revendication 1, dans laquelle l'émulsion est non-irritante pour les tissus oculaires.

10. Procédé de préservation et de stabilisation d'une émulsion pour usage pharmaceutique, comprenant l'addition à l'émulsion d'une quantité stabilisatrice et préservatrice d'un composé plurifonctionnel de formule (I), 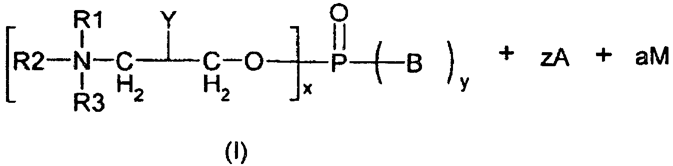 dans laquelle :
x est égal à 1 à 3 ou des mélanges correspondants ;
x+y est égal à 3 ;
a est égal à 0 à 2 ;
z est égal à x ;
B est O- ou OM ;
A est un anion ;
M est un cation ;
Y est choisi dans le groupe constitué d'OH, O-C₁-C₁₀ alkyle et O-C₁-C₁₀ alcényle ;
et
R1, R2 et R3 sont identiques ou différents et sont des groupes alkyle, alkyle substitués, alkyl aryle ou alcényle, éventuellement interrompus par-NHC(=O)-, pouvant atteindre 16 atomes de carbone, à condition que le total des atomes de carbone dans R1+R2+R3 soit compris entre 10 et 24, dans lequel la quantité du composé de formule I est comprise entre 0,05 et 0,2%.

11. Procédé selon la revendication 10, dans lequel :
R1 et R2 sont indépendamment un C₁-C₆ alkyle ;
R3 est un C₆-C₁₆ alkyle, éventuellement interrompu par -NHC(=O)-;
x est égal à 2 ;
a est égal à 1 ;
B est O⁻;
A est un halo ;
Y est choisi dans le groupe constitué de OH, O-C₁-C₁₀ alkyle et O-C₁-C₁₀ alcényle ; et
M est choisi dans le groupe constitué de sodium et de potassium.

12. Procédé selon la revendication 11, dans lequel :
R1 est un méthyle ;
R2 est un méthyle ;
R3 est un -(CH2)₁₁CH₃;
A est Cl⁻ ;
YestOH;
et
M est Na⁺.

13. Procédé selon la revendication 10, dans lequel l'émulsion comprend en outre un agent thérapeutique.

14. Procédé selon la revendication 10, dans lequel l'émulsion comprend en outre un agent émollient choisi dans le groupe constitué d'HPMC, HEC, CMC, gomme de Guar et gomme Xanthane.

15. Procédé de préparation d'une émulsion adaptée pour administration ophtalmique, otique ou nasale, dans lequel, à une composition comprenant un agent tampon, une huile, un émulsifiant, un ajusteur de pH et éventuellement un agent thérapeutique est ajouté, dans une quantité suffisante pour préserver et stabiliser ladite composition, un composé synthétique plurifonctionnel de formule (I) 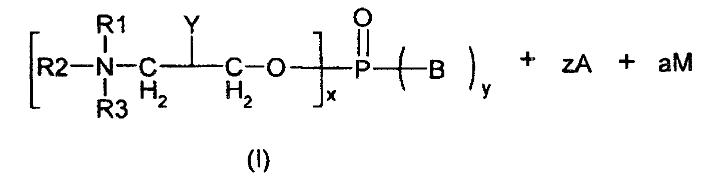 dans laquelle :
x est égal à 1 à 3 ou des mélanges correspondants ;
x+y est égal à 3 ;
a est égal à 0 à 2 ;
z est égal à x ;
B est O- ou OM ;
A est un anion ;
M est un cation ;
Y est choisi dans le groupe constitué d'OH, O-C₁-C₁₀ alkyle et O-C₁-C₁₀ alcényle ;
et
R1, R2 et R3 sont identiques ou différents et sont des groupes alkyle, alkyle substitués, alkyl aryle ou alcényle, éventuellement interrompus par - NHC(=O)-, pouvant atteindre 16 atomes de carbone, à condition que le total des atomes de carbone dans R1+R2+R3 soit compris entre 10 et 24, dans lequel la quantité du composé de formule I est comprise entre 0,05 et 0,2%.

16. Procédé selon la revendication 15, dans lequel :
R1 et R2 sont des méthyles
R3 est choisi dans le groupe constitué de (CH₂)₁₁CH₃, (CH₂)₃-NHC(=O)-(CH₂)₁₀CH₃, et -(CH₂)₃-NHC(=O)-(CH₂)₁₂CH₃;
x est égal à 2 ;
a est égal à 1 ;
B est O⁻;
A est un chloro ;
Y est OH, et
M est un ion de métal alcalin.

17. Procédé selon la revendication 16, dans lequel :
R1 est un méthyle ;
R2 est un méthyle ;
R3 est un -(CH₂)₁₁CH₃;
Y est OH ;
et
M est Na⁺.

18. Procédé selon la revendication 15, dans lequel l'émulsion comprend en outre un agent émollient choisi dans le groupe constitué d'HPMC, HEC, CMC, gomme de Guar et gomme Xanthane.

19. Procédé selon la revendication 15, dans lequel l'agent thérapeutique optionnel, lorsqu'il est présent, est choisi dans le groupe constitué d'agents ophtalmiques, otiques et nasaux.
